# EUROPEAN PATENT APPLICATION

(11) **EP 4 768 498 A1**
(43) Date of publication of application: **01.07.2026**
(21) Application number: 24859653.8
(22) Date of filing: 23.08.2024
(51) Int. Cl.: C07K 7/06, C07K 1/02, C07K 1/14, C07K 14/00, C07K 14/78, C12N 15/12

(54) **PEPTIDE, DROPLET FORMING KIT, DROPLET FORMING METHOD, HYDROPHOBIC SUBSTANCE CONCENTRATION METHOD, AND PROTEIN FOLDING METHOD**

(30) Priority: 25.08.2023 JP 2023137002; 14.05.2024 JP 2024078820
(71) Applicant: National University Corporation Tokyo University Of Agriculture and Technology, Fuchu-shi Tokyo 183-8538 (JP)
(72) Inventor: MURAOKA Takahiro, Fuchu-shi, Tokyo 183-8538 (JP); YAMASHITA Yukino, Fuchu-shi, Tokyo 183-8538 (JP)
(74) Representative: Cabinet Becker et Associés
(86) International application number: PCT/JP2024/030042
(87) International publication number: WO 2025/047620

(57) **Abstract**

A peptide has the following characteristics (a) to (c): (a) dissolving the peptide at an amount of 2 g or more at 20°C in 100 mL of a 50 mM Tris-HCl buffer (pH: 7.5) containing 0.3 M sodium chloride; (b) undergoing a reversible phase transition in the Tris-HCl buffer, in which a transition temperature when 2 g of the peptide is dissolved in 100 mL of the Tris-HCl buffer is 40°C to 70°C; and (c) forming droplets under a temperature condition at or above the transition temperature when 2 g of the peptide is dissolved in 100 mL of the Tris-HCl buffer.

## Description

### TECHNICAL FIELD

The present invention relates to a peptide, a droplet forming kit, a droplet forming method, a hydrophobic substance concentration method, and a protein folding method.

Priority is claimed on Japanese Patent Application No. 2023-137002 filed on August 25, 2023, and Japanese Patent Application No. 2024-078820 filed on May 14, 2024, the contents of which are incorporated herein by reference.

### BACKGROUND ART

An aqueous two-phase system (liquid-liquid phase separation) in which an aqueous solution spontaneously separates into two phases is a phenomenon useful for concentration and separation of solutes. As substances that form the aqueous two-phase system, polymers such as polyethylene glycol are well known. However, since the polymer has a high molecular weight, the viscosity of the aqueous solution increases. In addition, there are problems such as non-uniformity of a sample derived from the molecular weight distribution of the polymer and lot-to-lot variations.

Elastin-like peptides (ELPs) are known as a polypeptide that forms an aqueous two-phase system at high temperatures (Non-Patent Document 1). The ELPs have an amino acid sequence of (VPGXG)ₙ (X is an amino acid residue other than proline, and n is an integer of 1 or more). As the ELPs, ELPs having n > 40 are commonly used.

As applications of the ELPs, recovery of heavy metal ions using formation of the aqueous two-phase system (Non-Patent Document 2), separation using formation of an aqueous two-phase system of a protein having an ELP sequence introduced at a terminus (Non-Patent Document 3), and use in a drug delivery system by covalently bonding a low-molecular drug such as an anticancer agent to a terminus of an ELP (Non-Patent Document 4), and the like have been reported.

In addition, based on ELP sequences, development has been conducted of short-chain peptides made only of natural amino acids that form an aqueous two-phase system (Non-Patent Documents 1, 5, and 6).

### Citation List

### Non-Patent Documents

Non-Patent Document 1: Shogo Sumiyoshi, et al. Development of truncated elastin-like peptide analogues with improved temperature-response and self-assembling properties. Scientific Reports, 2022, 12, 19414.
Non-Patent Document 2: Shogo Sumiyoshi, et al. Metal ion scavenging activity of elastin-like peptide analogues containing a cadmium ion binding sequence. Scientific Reports, 2022, 12, 1861.
Non-Patent Document 3: Agnes Yeboah, et al. Elastin-like polypeptides: A strategic fusion partner for biologics. Biotechnology and Bioengineering, 2016, 113, 1617.
Non-Patent Document 4: Jose Carlos Rodriguez-Cabello, et al. Elastin-like polypeptides in drug delivery. Advanced Drug Delivery Reviews, 2016, 97, 85.
Non-Patent Document 5: Woo-jin Jeong, et al. Modular Self-Assembling Peptide Platform with a Tunable Thermoresponsiveness via a Single Amino Acid Substitution. Advanced Functional Materials, 2018, 28, 1803114.
Non-Patent Document 6: Suguru Taniguchi, et al. Development of short and highly potent self-assembling elastin-derived pentapeptide repeats containing aromatic amino acid residues. Journal of Peptide Science, 2016, 22, 36.

### SUMMARY OF INVENTION

### Technical Problem

In the related art, high-molecular-weight polymers such as polyethylene glycol, which have been used for forming the aqueous two-phase system, have problems such as high viscosity when formed into an aqueous solution and non-uniformity in molecular weight distribution. When the aqueous two-phase system can be formed using a low-molecular-weight material instead of the high-molecular-weight polymer, the problems caused by the use of high-molecular-weight polymers can be resolved.

Short-chain peptides that form the aqueous two-phase system based on ELP sequences have been developed, but such peptides have low water solubility, making it difficult to prepare an aqueous solution at a concentration sufficient for droplet formation. In addition, there are no known materials that enable concentration and recovery of proteins using the aqueous two-phase system.

Furthermore, peptides capable of promoting folding of proteins into native structures are expected to be applicable to the production of protein pharmaceuticals and the like.

Accordingly, the present invention provides a peptide usable for protein concentration and recovery or for protein folding, a droplet forming kit containing the peptide, a droplet forming method using the peptide, a hydrophobic substance concentration method, and a protein folding method.

### Solution to Problem

The present invention includes the following aspects.
[1] A peptide having the following characteristics (a) to (c):
   (a) dissolving the peptide at an amount of 2 g or more at 20°C in 100 mL of a 50 mM Tris-HCl buffer (pH: 7.5) containing 0.3 M sodium chloride;
   (b) undergoing a reversible phase transition in the Tris-HCl buffer, in which a transition temperature when 2 g of the peptide is dissolved in 100 mL of the Tris-HCl buffer is 40°C to 70°C; and
   (c) forming droplets under a temperature condition at or above the transition temperature when 2 g of the peptide is dissolved in 100 mL of the Tris-HCl buffer.
[2] The peptide according to [1], in which the peptide includes an amino acid sequence set forth in SEQ ID NO: 1,
   VPGXG (SEQ ID NO: 1)
   [in the formula, V represents a valine residue, P represents a proline residue, G represents a glycine residue, and X represents any amino acid residue other than a proline residue].
[3] The peptide according to [2], in which, in addition to the amino acid sequence set forth in SEQ ID NO: 1, the peptide includes one or more hydrophobic aliphatic amino acid residues at at least one of an N-terminus and a C-terminus.
[4] The peptide according to [3], in which the hydrophobic aliphatic amino acid residue is at least one selected from the group consisting of a leucine residue, an isoleucine residue, a valine residue, an alanine residue, and a methionine residue.
[5] The peptide according to [3], in which the peptide includes an amino acid sequence represented by General Formula (I),

   (Z1)ₙ₁-(VPGXG)ₘ-(Z2)ₙ₂ (I)

   [in the formula, Z1 and Z2 each independently represent a hydrophobic aliphatic amino acid residue, V represents a valine residue, P represents a proline residue, G represents a glycine residue, and X represents any amino acid residue other than a proline residue, n1 and n2 each independently represent an integer of 0 to 3, in which n1 + n2 ≥ 1, and m represents an integer of 3 to 5].
[6] The peptide according to [5], in which the amino acid sequence represented by General Formula (I) is an amino acid sequence set forth in SEQ ID NO: 2,
   LL-(VPGXG)₄ (SEQ ID NO: 2)
   [in the formula, L represents a leucine residue, V represents a valine residue, P represents a proline residue, G represents a glycine residue, and X represents any amino acid residue other than a proline residue].
[7] The peptide according to any one of [1] to [6], in which the peptide is used for concentrating a hydrophobic substance.
[8] The peptide according to [7], in which the hydrophobic substance is a denatured protein.
[9] The peptide according to any one of [1] to [8], in which the peptide includes at least one cysteine residue.
[10] The peptide according to [9], in which the peptide includes an amino acid sequence represented by General Formula (II),

   (Z1)ₙ₁-(Cx)ₚ₁-(VPGXG)ₘ₁-(Cy)ₚ₂-(VPGXG)ₘ₂-(Cx)ₚ₃-(Z2)ₙ₂ (II)

   [in the formula, Z1 and Z2 each independently represent a hydrophobic aliphatic amino acid residue, V represents a valine residue, P represents a proline residue, G represents a glycine residue, and X represents any amino acid residue other than a proline residue, Cx, Cy, and Cz each independently represent 1 to 5 amino acid residues including at least one cysteine residue, n1 and n2 each independently represent an integer of 0 to 3, in which n1 + n2 ≥ 1, m1 and m2 each independently represent an integer of 0 to 5, in which 3 ≤ m1 + m2 ≤ 5, and p1, p2, and p3 each independently represent 0 or 1, in which p1 + p2 + p3 ≥ 1].
[11] The peptide according to [10], in which the amino acid sequence represented by General Formula (II) is an amino acid sequence set forth in SEQ ID NO: 4,
   LL-(VPGXG)₂-CGHC-(VPGXG)₂ (SEQ ID NO: 4)
   [in the formula, L represents a leucine residue, V represents a valine residue, P represents a proline residue, G represents a glycine residue, C represents a cysteine residue, H represents a histidine residue, and X represents any amino acid residue other than a proline residue].
[12] The peptide according to any one of [1] to [11], in which the peptide is used for protein folding.
[13] A droplet forming kit including: the peptide according to any one of [1] to [12].
[14] A droplet forming method including: (A) a step of producing an aqueous solution containing the peptide according to any one of [1] to [6] and [9] to [11]; and (B) a step of incubating the aqueous solution at a temperature higher than the transition temperature of the peptide.
[15] A hydrophobic substance concentration method including: (A1) a step of producing an aqueous solution containing the peptide according to any one of [1] to [6] and [9] to [11] and a hydrophobic substance; and (B1) a step of incubating the aqueous solution at a temperature higher than the transition temperature of the peptide.
[16] The hydrophobic substance concentration method according to [15], in which the hydrophobic substance is a denatured protein.
[17] A protein folding method including: (A2) a step of producing an aqueous solution containing the peptide according to any one of [9] to [11] and a protein; and (B2) a step of incubating the aqueous solution.
[18] The protein folding method according to [17], in which the protein is a denatured protein.

### Advantageous Effects of Invention

According to the present invention, there are provided a peptide usable for protein concentration and recovery or for protein folding, a droplet forming kit containing the peptide, a droplet forming method using the peptide, a hydrophobic substance concentration method, and a protein folding method.

### BRIEF DESCRIPTION OF DRAWINGS

[FIG. 1] An RP-HPLC chart of LL-ELP4 synthesized in Experimental Example 1.
[FIG. 2] A MALDI-TOF-MS spectrum of LL-ELP4 synthesized in Experimental Example 1.
[FIG. 3] Results of monitoring transmittance changes with temperature in (a) a 1% by mass solution of FF-ELP4 and (b) a 2% by mass solution of LL-EPL4 prepared in Experimental Example 1; photographs showing the state of the solution at or below the transition temperature and photographs showing the state of the solution at or above the transition temperature are also shown.
[FIG. 4] Results of monitoring transmittance changes with temperature in a 5% by mass solution of LL-EPL4 prepared in Experimental Example 1.
[FIG. 5] Phase-contrast micrographs at 60°C of (a) a 1% by mass solution of FF-ELP4 and (b) a 2% by mass solution of LL-EPL4 prepared in Experimental Example 1.
[FIG. 6] An RP-HPLC chart of LL-ELP4-SS synthesized in Experimental Example 2.
[FIG. 7] A MALDI-TOF-MS spectrum of LL-ELP4 synthesized in Experimental Example 2.
[FIG. 8] Phase-contrast micrographs at 60°C of a mixed peptide solution of LL-ELP4 (2% by mass)/LL-ELP4-SS (0.16% by mass) prepared in Experimental Example 2.
[FIG. 9] Results of a folding test of reduced and denatured BPTI in a mixed peptide solution of LL-ELP4 (2% by mass)/LL-ELP4-SS (0.16% by mass) prepared in Experimental Example 2.
[FIG. 10] Phase-contrast micrographs at 60°C of a 2% by mass solution of LL-ELP4-SS prepared in Experimental Example 2.
[FIG. 11] Results of a folding test of reduced and denatured BPTI in a 2% by mass solution of LL-ELP4-SS prepared in Experimental Example 2.

### DESCRIPTION OF EMBODIMENTS

A numerical range represented by "to" refers to a range including numerical values denoted before and after "to" as a lower limit value and an upper limit value.

The term "include (comprise)" means that additional elements other than specified elements may be included. The term "consist of" means that elements other than specified elements are not included. The term "consist essentially of" means that elements other than specified elements are not included in an aspect that exhibits a special function (such as an aspect that completely loses the effect of the invention). In the present specification, the description "comprise" includes the aspect of "consist of" and the aspect of "consist essentially of".

The term "peptide" refers to a polymer of amino acids bonded by amide bonds. Unless otherwise specified, the peptides are described by generally accepted one-letter codes or three-letter codes. Unless otherwise specified, an amino acid sequence of the peptide is described from the N-terminal side to the C-terminal side.

The peptide may be a polymer of natural amino acids, a polymer of natural amino acids and non-natural amino acids, or a polymer of non-natural amino acids. From the viewpoint of biocompatibility and biodegradability, the peptide is preferably a polymer of natural amino acids, and preferably does not include non-natural amino acid residues.

The "natural amino acid" is an amino acid existing in nature. The natural amino acid is preferably selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine.

The "non-natural amino acid" is an amino acid that does not exist in nature. Examples of the non-natural amino acid include modified derivatives of natural amino acids.

### <Peptide>

A first aspect of the present invention is a peptide having the following characteristics (a) to (c) (hereinafter, also referred to as "peptide (P)"):
(a) dissolving the peptide at an amount of 2 g or more at 20°C in 100 mL of a 50 mM Tris-HCl buffer (pH: 7.5) containing 0.3 M sodium chloride;
(b) undergoing a reversible phase transition in the Tris-HCl buffer, in which a transition temperature when 2 g of the peptide is dissolved in 100 mL of the Tris-HCl buffer is 40°C to 70°C; and
(c) forming droplets under a temperature condition at or above the transition temperature when 2 g of the peptide is dissolved in 100 mL of the Tris-HCl buffer.

In one embodiment, the peptide (P) is used for forming droplets by an aqueous two-phase system. In the aqueous two-phase system, an aqueous solution spontaneously separates into two phases. In one embodiment, in an aqueous solution in which the peptide (P) is dissolved, the aqueous solution separates into two phases due to aggregation of the peptide (P), and droplets are formed.

By having the above-described characteristics (a) to (c), the peptide (P) does not have an excessively high viscosity when formed into an aqueous solution, and can form droplets of an appropriate size under relatively mild conditions. In addition, a hydrophobic substance (for example, a denatured protein) can be concentrated in the droplets. Furthermore, among the peptides (P), those having a cysteine residue can promote protein folding.

### (Regarding (a))

The 50 mM Tris-HCl buffer (pH: 7.5) containing 0.3 M sodium chloride in (a) described above (hereinafter, also referred to as "NaCl-containing Tris-HCl (pH: 7.5)") can be produced by dissolving sodium chloride in a 50 mM Tris-HCl buffer (pH: 7.5) to a final concentration of 0.3 M. The 50 mM Tris-HCl buffer (pH: 7.5) can be produced, for example, by dissolving 0.6057 g of trishydroxymethylaminomethane in 50 to 80 mL of water (distilled water or the like), adjusting the pH to 7.5 with hydrochloric acid, and then adding water to make the total volume 100 mL.

The peptide (P) dissolves in an amount of 2 g or more at 20°C in 100 mL of the NaCl-containing Tris-HCl (pH: 7.5). From the viewpoint of forming droplets under relatively mild conditions, it is preferable not to dissolve in an amount of 20 g or more at 20°C in 100 mL of the NaCl-containing Tris-HCl (pH: 7.5).

The maximum dissolution amount of the peptide (P) in 100 mL of the NaCl-containing Tris-HCl (pH: 7.5) at 20°C is preferably 3 g or more, and more preferably 5 g or more. The above-described maximum dissolution amount is preferably 20 g or less, and more preferably 15 g or less. The maximum dissolution amount of the peptide according to the present embodiment in 100 mL of the NaCl-containing Tris-HCl (pH: 7.5) at 20°C is preferably 2 to 15 g.

The maximum dissolution amount of the peptide (P) in 100 mL of the NaCl-containing Tris-HCl (pH: 7.5) at 20°C can be confirmed, for example, as follows. 100 mL of the NaCl-containing Tris-HCl (pH: 7.5) is charged into a container (beaker, flask, or the like), and the peptide (P) is added thereto while maintaining the temperature at 20°C using a water bath, an ultrasonic cleaner, or the like. The addition of the peptide (P) is preferably performed while stirring the NaCl-containing Tris-HCl (pH: 7.5) with a stirrer or the like. The peptide (P) is added little by little and dissolved, and when dissolution of the entire amount of the added peptide (P) is confirmed, the peptide (P) is further added. The amount of the peptide (P) added until immediately before the newly added peptide (P) stops dissolving in 100 mL of the NaCl-containing Tris-HCl (pH: 7.5) can be defined as the maximum dissolution amount of the peptide (P).

### (Regarding (b))

The Tris-HCl buffer in (b) is the same as the NaCl-containing Tris-HCl (pH: 7.5) in (a). The peptide (P) undergoes a reversible phase transition in a peptide solution produced by dissolving the peptide (P) in the NaCl-containing Tris-HCl (pH: 7.5).

The "phase transition" refers to a change from a state in which liquid-liquid phase separation has not occurred to a state in which liquid-liquid phase separation has occurred, or vice versa. The "transition temperature" refers to the temperature at which the phase transition occurs.

In the peptide solution produced by dissolving 2 g of the peptide (P) in 100 mL of the NaCl-containing Tris-HCl (pH: 7.5) (hereinafter, also referred to as "peptide (P) solution"), liquid-liquid phase separation occurs at a temperature equal to or higher than the transition temperature to form an aqueous two-phase system. At a temperature at or below the transition temperature, the liquid-liquid phase separation is eliminated, resulting in a homogeneous solution. The peptide (P) solution reversibly changes between an aqueous single-phase system state and an aqueous two-phase system state with the transition temperature as a boundary.

When the peptide (P) solution undergoes the liquid-liquid phase separation to form the aqueous two-phase system, droplets are formed in the peptide (P) solution, and a transmittance of the peptide (P) solution decreases. Therefore, whether the phase transition occurs can be determined from changes in the transmittance of the peptide (P) solution. The transmittance of the peptide (P) solution can be confirmed, for example, by measuring absorbance at 600 nm using a spectrophotometer.

The transition temperature of the peptide (P) solution is 40°C to 70°C. The transition temperature is preferably 45°C to 65°C, more preferably 50°C to 60°C, and still more preferably 50°C to 55°C.

### (Regarding (c))

The Tris-HCl buffer in (c) is the same as the NaCl-containing Tris-HCl (pH: 7.5) in (a). The peptide (P) forms droplets at a temperature at or above the transition temperature in the peptide solution produced by dissolving the peptide (P) in the NaCl-containing Tris-HCl (pH: 7.5).

The "transition temperature" is the same as the "transition temperature" described in (b) above, and is the temperature at which the phase transition occurs. In the peptide (P) solution produced by dissolving 2 g of the peptide (P) in 100 mL of the NaCl-containing Tris-HCl (pH: 7.5), the liquid-liquid phase separation occurs at a temperature at or above the transition temperature to form an aqueous two-phase system. When the peptide (P) solution undergoes the liquid-liquid phase separation to form an aqueous two-phase system, droplets are formed in the peptide (P) solution.

The formation of droplets can be confirmed, for example, by observation with a phase-contrast microscope, measurement of transmittance with a spectrophotometer (for example, absorbance measurement at 600 nm), or the like.

### (Specific examples of peptide (P))

Specific examples of the peptide (P) include a peptide including an amino acid sequence set forth in SEQ ID NO: 1 described below (hereinafter, also referred to as "amino acid sequence (1)").
VPGXG (SEQ ID NO: 1)
[in the formula, V represents a valine residue, P represents a proline residue, G represents a glycine residue, and X represents any amino acid residue other than a proline residue].

In the amino acid sequence (1), examples of X include amino acid residues derived from amino acids selected from the group consisting of alanine, arginine, asparagine, aspartic acid, cysteine, glutamine, glutamic acid, glycine, histidine, isoleucine, leucine, lysine, methionine, phenylalanine, serine, threonine, tryptophan, tyrosine, and valine. Among the above, X is preferably an alanine residue, a valine residue, a leucine residue, an isoleucine residue, or a methionine residue, and more preferably a valine residue.

It is preferable that the peptide (P) includes one or more hydrophobic aliphatic amino acid residues at at least one of an N-terminus and a C-terminus, in addition to the amino acid sequence (1).

The "hydrophobic aliphatic amino acid residue" is an amino acid residue derived from a hydrophobic amino acid including no aromatic ring.

Specific examples of the hydrophobic aliphatic amino acid residue include at least one selected from the group consisting of a leucine residue, an isoleucine residue, a valine residue, an alanine residue, and a methionine residue. Among the above, from the viewpoint of hydrophobicity, a leucine residue, an isoleucine residue, or a valine residue is preferable, and a leucine residue is more preferable.

The number of hydrophobic aliphatic amino acid residues added to at least one of the N-terminus and the C-terminus is preferably 1 to 5, more preferably 2 to 4, and still more preferably 2 or 3. When the number of amino acid residues added to the N-terminus and/or the C-terminus is within the above-described preferred range, droplets of appropriate size are easily formed. When a plurality of hydrophobic aliphatic amino acid residues are added, the plurality of hydrophobic aliphatic amino acid residues may be the same amino acid residue or different amino acid residues.

The hydrophobic aliphatic amino acid residue may be added to the N-terminus, may be added to the C-terminus, or may be added to both the N-terminus and the C-terminus. When the hydrophobic aliphatic amino acid residues are added to both the N-terminus and the C-terminus, the type and number of hydrophobic aliphatic amino acid residues added to the N-terminus and the C-terminus may be the same or different. For example, the above-described number may be one at each of the N-terminus and the C-terminus, two at the N-terminus and one at the C-terminus, or one at the N-terminus and two at the C-terminus.

In one embodiment, the peptide (P) is preferably a peptide consisting of an amino acid sequence represented by General Formula (I) (hereinafter, also referred to as "peptide (P-I)").

(Z1)ₙ₁-(VPGXG)ₘ-(Z2)ₙ₂ (I)

[in the formula, Z1 and Z2 each independently represent a hydrophobic aliphatic amino acid residue, V represents a valine residue, P represents a proline residue, G represents a glycine residue, and X represents any amino acid residue other than a proline residue, n1 and n2 each independently represent an integer of 0 to 3, in which n1 + n2 ≥ 1, and m represents an integer of 3 to 5]

In Formula (1), examples of the hydrophobic aliphatic amino acid residue for Z1 and Z2 include the same as those described above. As the hydrophobic aliphatic amino acid residue, a leucine residue, an isoleucine residue, or a valine residue is preferable, and a leucine residue is more preferable.

In Formula (I), n1 and n2 are preferably 1 ≤ n1 + n2 ≤ 4, and more preferably 2 ≤ n1 + n2 ≤ 3.

It is preferable that n1 is 2 or 3 and n2 is 0, or n1 is 0 and n2 is 2 or 3; it is more preferable that n1 is 2 or 3 and n2 is 0; and it is still more preferable that n1 is 2 and n2 is 0.

When n1 is an integer of 2 or more, two or more Z1's may be the same or different from each other; and when n2 is an integer of 2 or more, two or more Z2's may be the same or different from each other.

Examples of the arbitrary amino acid residue for X include the same as those described above. X is preferably a valine residue.

In Formula (I), m is preferably 3 or 4, and more preferably 4.

In one embodiment, the peptide (P-I) preferably consists of an amino acid sequence set forth in SEQ ID NO: 2.
LL-(VPGXG)₄ (SEQ ID NO: 2)
[in the formula, L represents a leucine residue, V represents a valine residue, P represents a proline residue, G represents a glycine residue, and X represents any amino acid residue other than a proline residue]

Examples of X in the amino acid sequence set forth in SEQ ID NO: 2 include the same as those described above, and a valine residue is preferable.

In one embodiment, the peptide (P-I) preferably consists of an amino acid sequence set forth in SEQ ID NO: 3.
LL-(VPGVG)₄ (SEQ ID NO: 3)
[in the formula, L represents a leucine residue, V represents a valine residue, P represents a proline residue, and G represents a glycine residue]

The peptide (P-I) forms droplets by liquid-liquid phase separation when dissolved in water or a buffer in an appropriate amount to form an aqueous solution and brought to a temperature at or above the transition temperature. When a hydrophobic substance is allowed to coexist in the above-described aqueous solution, the hydrophobic substance is selectively incorporated into the droplets. The droplets can be separated and recovered by centrifugation or the like. The hydrophobic substance incorporated into the droplets can be recovered by disrupting the droplets. Therefore, the peptide (PI) can be used for concentrating the hydrophobic substance. Alternatively, the peptide (P-I) can be used for separating and recovering the hydrophobic substance.

Therefore, the peptide (P-I) can be used as a concentrating agent for a hydrophobic substance or an extracting agent for a hydrophobic substance.

Examples of the hydrophobic substance include denatured proteins. The "denatured protein" is a protein in which a tertiary structure has undergone a change due to physical or chemical action. Examples of the denatured protein include reduced denatured proteins in which disulfide bonds have been reduced by a reducing agent or the like.

In one embodiment, the peptide (P) may be a peptide including at least one cysteine residue (hereinafter, also referred to as "peptide (P-11)"). The number of cysteine residues of the peptide (P-II) includes, for example, 1 to 10, and may be 1 to 8, 1 to 7, 1 to 6, 1 to 5, 1 to 4, 1 to 3, 1 or 2.

For example, the cysteine residue in the peptide (P-II) may be located at the N-terminus, at the C-terminus, or within the peptide. When the peptide (P-II) has the amino acid sequence (1), the cysteine residue may be present, for example, on either the N-terminal side or the C-terminal side of the amino acid sequence (1), or may be present on both the N-terminal side and the C-terminal side of the amino acid sequence (1). When the peptide (P-II) has two or more amino acid sequences (1), the cysteine residue may be present between the plurality of amino acid sequences (1).

When the peptide (P-II) has the amino acid sequence (1) and the hydrophobic aliphatic amino acid residue at the N-terminus and/or C-terminus, the cysteine residue may be present, for example, between the amino acid sequence (1) and the hydrophobic aliphatic amino acid residue.

In one embodiment, the peptide (P-II) is preferably a peptide consisting of an amino acid sequence represented by General Formula (II).

(Z1)ₙ₁-(Cx)ₚ₁-(VPGXG)ₘ₁-(Cy)ₚ₂-(VPGXG)ₘ₂-(Cx)ₚ₃-(Z2)ₙ₂ (II)

[in the formula, Z1 and Z2 each independently represent a hydrophobic aliphatic amino acid residue, V represents a valine residue, P represents a proline residue, G represents a glycine residue, and X represents any amino acid residue other than a proline residue, Cx, Cy, and Cz each independently represent 1 to 5 amino acid residues including at least one cysteine residue, n1 and n2 each independently represent an integer of 0 to 3, in which n1 + n2 ≥ 1, m1 and m2 each independently represent an integer of 0 to 5, in which 3 ≤ m1 + m2 ≤ 5, and p1, p2, and p3 each independently represent 0 or 1]

In Formula (11), Z1, Z2, n1, n2, and X are the same as Z1, Z2, n1, n2, and X in Formula (I) described above.

In Formula (II), m1 and m2 are preferably 0 to 4, more preferably 1 to 4, still more preferably 2 or 3, and particularly preferably 2. Examples of the combination of m1 and m2 include m2 = 5, 4, or 3 for m1 = 0; m2 = 4, 3, or 2 for m1 = 1; m2 = 3, 2, or 1 for m1 = 2; m2 = 2, 1, or 0 for m1 = 3; m2 = 1 or 0 for m1 = 4; and m2 = 0 for m1 = 5. In one embodiment, m1 is 2 and m2 is 2.

In Formula (II), examples of the number of cysteine residues in Cx, Cy, and Cz include 1 to 5, 1 to 4, 1 to 3, 1, or 2. The amino acid residues other than the cysteine residue in Cx, Cy, and Cz are not particularly limited, and examples thereof include a histidine residue, an aspartic acid residue, a glutamic acid residue, a lysine residue, an arginine residue, a serine residue, a threonine residue, an asparagine residue, a glutamine residue, a tyrosine residue, a glycine residue, an alanine residue, a proline residue, a valine residue, a leucine residue, an isoleucine residue, a methionine residue, a phenylalanine residue, a tryptophan residue, an ornithine residue, and a selenocysteine residue. The amino acid residues other than the cysteine residue in Cx, Cy, and Cz may be, for example, a hydrophilic amino acid residue. Examples of the hydrophilic amino acid residue include a histidine residue, an aspartic acid residue, a glutamic acid residue, a lysine residue, an arginine residue, a serine residue, a threonine residue, an asparagine residue, a glutamine residue, a tyrosine residue, an ornithine residue, and a selenocysteine residue. The amino acid residues other than the cysteine residue in Cx, Cy, and Cz may be, for example, a hydrophobic amino acid residue. Examples of the hydrophobic amino acid residue include a glycine residue, an alanine residue, a valine residue, a methionine residue, a leucine residue, an isoleucine residue, a phenylalanine residue, a tryptophan residue, and a proline residue.

Examples of the number of amino acid residues in Cx, Cy, and Cz include 1 to 4, 1 to 3, 1 to 2, or 1. Examples of Cx, Cy, and Cz include an amino acid sequence represented by General Formula (III).

(X¹)_{q1}C(X²)_{q2}C(X³)_{q3} (III)

[in the formula, X¹, X², and X³ each independently represent any amino acid residue, and q1, q2, and q3 each independently represent an integer of 0 to 4]

In Formula (III), examples of X¹, X², and X³ include amino acid residues derived from amino acids selected from the group consisting of alanine, arginine, asparagine, aspartic acid, cysteine, glutamine, glutamic acid, glycine, histidine, isoleucine, leucine, lysine, methionine, phenylalanine, serine, threonine, tryptophan, tyrosine, proline, and valine. Among the above, X is preferably an amino acid residue selected from the group consisting of a glycine residue and a histidine residue.

In Formula (III), q2 is preferably an integer of 1 to 4, more preferably an integer of 1 to 3, and still more preferably 1 or 2.

In Formula (III), q1 and q3 are preferably an integer of 0 to 3, more preferably an integer of 0 to 2, and still more preferably 0 or 1.

Specific examples of Cx, Cy, and Cz include CGHC.

In Formula (II), p1, p2, and p3 may be, for example, all 1 (p1 = 1, p2 = 1, p3 = 1), any two may be 1 and the other one may be 1 (p1 = 1, p2 = 1, p3 = 0; p1 = 1, p2 = 0, p3 = 1; or p1 = 0, p2 = 1, p3 = 1), or any one may be 1 and the other two may be 0 (p1 = 1, p2 = 0, p3 = 0; p1 = 0, p2 = 1, p3 = 0; or p1 = 0, p2 = 0, p3 = 1). 1 ≤ p1 + p2 + p3 ≤ 2 is preferable, and p1 + p2 + p3 = 1 is more preferable.

In one embodiment, the peptide (P-II) preferably consists of an amino acid sequence set forth in SEQ ID NO: 4.
LL-(VPGXG)₂-CGHC-(VPGXG)₂ (SEQ ID NO: 4)
[in the formula, L represents a leucine residue, V represents a valine residue, P represents a proline residue, G represents a glycine residue, C represents a cysteine residue, H represents a histidine residue, and X represents any amino acid residue other than a proline residue]

Examples of X in the amino acid sequence set forth in SEQ ID NO: 4 include the same as those described above, and a valine residue is preferable.

In one embodiment, the peptide (P-II) preferably consists of an amino acid sequence set forth in SEQ ID NO: 5.
LL-(VPGVG)₂-CGHC-(VPGVG)₂ (SEQ ID NO: 5)
[in the formula, L represents a leucine residue, V represents a valine residue, P represents a proline residue, G represents a glycine residue, C represents a cysteine residue, and H represents a histidine residue]

Since the peptide (P-II) has a cysteine residue, the peptide can promote folding of a denatured protein. Examples of the denatured protein include a denatured protein in which a disulfide bond has been reduced.

The peptide (P-II) forms droplets by liquid-liquid phase separation when dissolved in water or a buffer in an appropriate amount to form an aqueous solution and brought to a temperature at or above the transition temperature. When a denatured protein is allowed to coexist in the above-described aqueous solution, the denatured protein is selectively incorporated into the droplets. The denatured protein is folded within the droplets to become a native structural protein. The folded protein moves out of the droplets. Therefore, the folded protein can be efficiently recovered by recovering the aqueous phase by centrifugation or the like.

Accordingly, the peptide (P-II) can be used for protein folding and can be used as a protein folding agent.

By using the peptide (P-II) in combination with the peptide (P-I), droplet formation can be performed efficiently.

Since the peptide (P) does not have very high hydrophobicity, preparation of the aqueous solution is easy. In addition, since the peptide (P) is a low-molecular-weight molecule, the viscosity of the aqueous solution does not become too high, and there is no problem of molecular weight distribution. Moreover, the peptide (P) can be produced by a general peptide solid-phase synthesis method. Furthermore, when composed only of natural amino acid residues, the peptide (P) can be a peptide excellent in biocompatibility and biodegradability.

### <Droplet forming kit>

A second aspect of the present invention is a droplet forming kit including the peptide (P).

The peptide (P) included in the kit according to the present embodiment may be the peptide (P-II) or a peptide (P) that is not the peptide (P-II). As the peptide (P) that is not the peptide (P-II), the peptide (P-I) is preferable.

When the kit according to the present embodiment includes the peptide (P) that is not the peptide (P-II), the kit according to the present embodiment can be used as a kit for concentrating a hydrophobic substance or a kit for extracting a hydrophobic substance. When the kit according to the present embodiment includes the peptide (P-II), the kit according to the present embodiment can be used as a kit for protein folding. Examples of the hydrophobic substance include denatured proteins. Examples of the denatured proteins include denatured proteins in which a disulfide bond has been reduced.

When the kit according to the present embodiment includes the peptide (P-II), the kit may further include the peptide (P) that is not the peptide (P-II). For example, the kit according to the present embodiment may include the peptide (P-II) and the peptide (P-I).

The kit according to the present embodiment may include other configurations in addition to the peptide (P). Examples of other configurations include an aqueous solvent for dissolving the peptide (P).

Examples of the aqueous solvent include water (pure water or ultrapure water such as distilled water, ion exchange water, reverse osmosis water), and buffers. The buffer is not particularly limited, and those usually used for biochemical tests and the like can be used without particular limitation. Examples of the buffer include, but are not limited to, Tris-HCl buffer, phosphate buffer, phosphate buffered saline, acetate buffer, citrate buffer, and HEPES buffer.

Among the above, as the aqueous solvent, a Tris-HCl buffer is preferable, and a sodium chloride-containing Tris-HCl buffer is more preferable. Specific examples of the solvent include 50 mM Tris-HCl buffer (pH: 7.5) containing 0.3 M sodium chloride.

The kit according to the present embodiment may include a reagent for preparing the aqueous solvent. For example, when preparing the sodium chloride-containing Tris-HCl buffer as the aqueous solvent, the kit according to the present embodiment may include sodium chloride, trishydroxymethylaminomethane, and hydrochloric acid.

The kit according to the present embodiment may include a container (test tube, plastic tube, beaker, flask, microtube, and the like) used for preparing the peptide solution, an instruction, and the like.

The kit according to the present embodiment can be used for producing droplets.

### <Droplet forming method>

A third aspect of the present invention is a droplet forming method. The droplet forming method includes (A) a step of producing an aqueous solution containing the peptide (P), and (B) a step of incubating the aqueous solution at a temperature above the transition temperature of the peptide (P).

### (Step (A))

In the step (A), an aqueous solution containing the peptide (P) is produced.

The aqueous solution containing the peptide (P) can be produced by dissolving the peptide (P) in an aqueous solvent. The peptide (P) may be the peptide (P-II), may be the peptide (P) that is not the peptide (P-II), or may be a combination of the peptide (P-II) and the peptide (P) that is not the peptide (P-II). As the peptide (P) that is not the peptide (P-II), the peptide (P-I) is preferable.

Examples of the aqueous solvent include the same as those mentioned in the section of <Droplet forming kit> above. As the aqueous solvent, a Tris-HCl buffer is preferable, and a sodium chloride-containing Tris-HCl buffer is more preferable. Specific examples of the aqueous solvent include 50 mM Tris-HCl buffer (pH: 7.5) containing 0.3 M sodium chloride.

The peptide (P) is preferably dissolved in the aqueous solvent so as to have a concentration of 2% by mass or more. The concentration of the peptide (P) in the aqueous solution includes 2% to 15% by mass, preferably 2% to 10% by mass, more preferably 2% to 8% by mass, and still more preferably 2% to 5% by mass.

When the peptide (P) is difficult to dissolve, the peptide (P) may be dissolved in the aqueous solvent while heating the aqueous solvent using a water bath or the like. The peptide (P) may be dissolved while stirring the aqueous solvent using a stirrer, a vortex, an ultrasonic cleaner, or the like.

When the peptide (P-1I) and the peptide (P) that is not the peptide (P-1I) are used in combination, the concentration of the peptide (P) that is not the peptide (P-II) may be 2% by mass or more, and the concentration of the peptide (P-II) may be less than 2% by mass. In this case, the concentration of the peptide (P-II) can be, for example, 0.001% to 1.5% by mass, 0.01% to 1.0% by mass, 0.05% to 0.5% by mass, or 0.10% to 0.5% by mass.

### (Step (B))

In the step (B), the aqueous solution produced in the step (A) is incubated at a temperature above the transition temperature of the peptide (P).

For the incubation, an apparatus having a temperature maintaining function, such as an incubator, can be used.

Since the transition temperature varies depending on the amino acid sequence of the peptide (P), the incubation temperature can be set according to the transition temperature of the peptide (P). The incubation temperature can be set, for example, to a temperature 1°C to 10°C higher than the transition temperature of the peptide (P).

The incubation time may be a time sufficient for droplets to be formed by liquid-liquid phase separation. Examples of the incubation time include 10 seconds or longer and 5 minutes or shorter. The incubation time may be 30 seconds or longer and 5 minutes or shorter, or 1 minute or longer and 5 minutes or shorter.

The particle size of the droplets formed by the droplet forming method according to the present embodiment is not particularly limited, and examples thereof include a particle size of 1 to 100 µm. The particle size of the droplets may be, for example, 5 to 80 µm, 10 to 60 µm, or 20 to 50 µm.

In the droplet forming method according to the present embodiment, since the peptide (P) is used, the viscosity of the aqueous solution does not become excessively high. In addition, unlike polymer materials such as polyethylene glycol, since there is no molecular weight distribution, the droplets can be formed with stable quality.

### <Hydrophobic substance concentration method>

A fourth aspect of the present invention is a hydrophobic substance concentration method. The method according to the present aspect includes (A1) a step of producing an aqueous solution containing the peptide (P) and a hydrophobic substance, and (B1) a step of incubating the aqueous solution at a temperature below the phase transition temperature of the peptide (P).

### (Step (A1))

In the step (A1), an aqueous solution containing the peptide (P) and a hydrophobic substance is produced.

It is preferable that the peptide (P) is not the peptide (P-II), and it is more preferable to be the peptide (P-I).

The hydrophobic substance is not particularly limited. Examples of the hydrophobic substance include denatured proteins, hydrophobic polymers, and hydrophobic drugs. The denatured protein is a protein as a native structural protein denatured by a denaturing agent. Examples of the denaturing agent include a reducing agent. Examples of the denatured protein include reduced denatured proteins in which one or more disulfide bonds are reduced.

The aqueous solution in the step (A1) can be produced by adding the hydrophobic substance to an aqueous solution containing the peptide (P).

The aqueous solution containing the peptide (P) can be produced by the same method as in the step (A) of <Droplet forming method> described above.

The hydrophobic substance may be added to the aqueous solution as it is, or may be dissolved in a suitable solvent and then added to the aqueous solution. The hydrophobic substance may be added to the aqueous solution as a mixture with another substance (for example, a hydrophilic substance). When the hydrophobic substance is a denatured protein, the denatured protein may be added to the aqueous solution as a mixture with a native protein.

A concentration of the hydrophobic substance in the aqueous solution is not particularly limited. The concentration of the hydrophobic substance is, for example, 1 to 10000 µM.

### (Step (B1))

In the step (B1), the aqueous solution produced in the step (A1) is incubated at a temperature above the transition temperature of the peptide (P).

The step (B1) can be performed in the same manner as in the step (B) of <Droplet forming method> described above. Since the hydrophobic substance is selectively incorporated into the droplets formed in the step (B1), the hydrophobic substance can be concentrated in the droplets.

### (Other steps)

The hydrophobic substance concentration method according to the present embodiment may include other steps in addition to the above-described steps (A1) and (B1). Examples of other steps include (C1) a step of recovering the droplets from the aqueous solution after the step (B1), and (D1) a step of recovering the hydrophobic substance from the droplets.

### <<Step (C1)>>

After the step (B1), the droplets may be recovered from the above-described aqueous solution.

The droplets can be recovered by centrifugation or the like. The rotation speed of the centrifugation is, for example, 5000 to 10000 rpm. The centrifugation time includes, for example, 10 seconds or longer, and may be 20 seconds or longer. From the viewpoint of preventing destruction of the droplets, the centrifugation time is preferably 5 minutes or shorter, more preferably 3 minutes or shorter, and still more preferably 1 minute or shorter. The centrifugation time may be 10 seconds or longer and 5 minutes or shorter, 20 seconds or longer and 3 minutes or shorter, or 20 seconds or longer and 1 minute or shorter. The droplets can be recovered as a precipitate of centrifugation.

### <<Step (D1)>>

The droplets recovered in the step (C1) may be disrupted to recover the hydrophobic substance. Since the hydrophobic substance is incorporated without aggregation in the droplets, the recovered hydrophobic substance can be used for various applications.

In the hydrophobic substance concentration method according to the present embodiment, the hydrophobic substance can be concentrated in the droplets while suppressing aggregation. Many protein pharmaceuticals, such as antibodies and insulin, form quaternary structures composed of multiple polypeptide chains. Promoting such quaternary structure formation is important for increasing the production efficiency of protein pharmaceuticals and reducing production cost. In order to form a quaternary structure, there is a need to concentrate multiple polypeptide chains while suppressing aggregation. In the hydrophobic substance concentration method according to the present embodiment, the hydrophobic substance such as a reduced and denatured protein can be concentrated within the droplets while suppressing aggregation. Therefore, the droplets may be used as a site where the multiple polypeptide chains form the quaternary structure. In addition, it is expected to contribute to the development of materials that promote quaternary structure formation.

In the hydrophobic substance concentration method according to the present embodiment, the hydrophobic substance is concentrated in the droplets and the droplets are recovered, whereby the hydrophobic substance can be separated and recovered.

### <Protein folding method>

A fifth aspect of the present invention is a protein folding method. The method according to the present aspect includes (A2) a step of producing an aqueous solution containing the peptide (P) and a protein, and (B2) a step of incubating the aqueous solution at a temperature above the transition temperature of the peptide.

### (Step (A2))

In the step (A2), an aqueous solution containing the peptide (P) and a protein is produced.

The peptide (P) is preferably the peptide (P-II). The peptide (P) may be a combination of the peptide (P-II) and the peptide (P) that is not the peptide (P-II). As the peptide (P) that is not the peptide (P-II), the peptide (P-I) is preferable.

The protein is a protein to be folded, and is preferably a denatured protein. The denatured protein is preferably a reduced and denatured protein in which one or more disulfide bonds have been reduced.

The protein may be an unfolded protein or a misfolded protein. The unfolded protein is a protein that is not folded. In the unfolded protein, disulfide bonds formed in a native structural protein are reduced to thiol groups. A protein reduced and denatured by a reducing agent is the unfolded protein.

The misfolded protein is a protein in which one or more disulfide bonds are formed, and has a structure other than a native structure. The native structural protein usually has a thermodynamically most stable structure. However, the misfolded protein is folded into a structure other than the native structure.

The protein may be a protein having at least one selected from the group consisting of a disulfide bond and a reduced disulfide bond.

The aqueous solution in the step (A2) can be produced by adding the protein to an aqueous solution containing the peptide (P).

The aqueous solution containing the peptide (P) can be produced by the same method as in the step (A) of <Droplet forming method> described above. The aqueous solution containing the peptide (P) can be produced by dissolving the peptide (P) in an aqueous solvent.

Examples of the aqueous solvent include the same as those mentioned in the section of <Droplet forming kit> above. As the aqueous solvent, a Tris-HCl buffer is preferable, and a sodium chloride-containing Tris-HCl buffer is more preferable. Specific examples of the aqueous solvent include 50 mM Tris-HCl buffer (pH: 7.5) containing 0.3 M sodium chloride.

The concentration of the peptide (P) in the aqueous solution containing the peptide (P) may be any concentration capable of promoting the protein folding, and includes, for example, 0.001% by mass or more. Examples of the concentration of the peptide (P) include 0.005% by mass or more, 0.01% by mass or more, 0.05% by mass or more, and 0.10% by mass or more.

When the peptide (P) is only the peptide (P-II), the concentration of the peptide (P) in the aqueous solution may be, for example, 2% by mass or less, 1.5% by mass or less, 1.0% by mass or less, or 0.5% by mass or less. In this case, the concentration of the peptide (P-II) in the aqueous solution may be, for example, 0.001 to 1.5% by mass, 0.01 to 1.0% by mass, 0.05 to 0.5% by mass, or 0.10 to 0.5% by mass.

When the peptide (P-II) and the peptide (P) that is not the peptide (P-II) are used in combination as the peptide (P), a concentration of the peptide (P) that is not the peptide (P-II) may be, for example, 2% by mass or more. Examples of the concentration of the peptide (P) that is not the peptide (P-1I) in the above-described aqueous solution include 2% to 15% by mass, preferably 2% to 10% by mass, more preferably 2% to 8% by mass, and still more preferably 2% to 5% by mass. In this case, examples of the concentration of the peptide (P-11) include the same concentrations as described above.

The protein may be added to the aqueous solution as it is, or may be dissolved in a suitable solvent and then added to the aqueous solution. The concentration of the protein in the aqueous solution is not particularly limited. Examples of the concentration of the protein include 1 to 10000 µM.

### (Step (B2))

In the step (B2), the aqueous solution produced in the step (A2) is incubated. The incubation temperature may be any temperature that permits protein folding. Examples of the incubation temperature include 10°C to 70°C, preferably 20°C to 65°C, and more preferably 30°C to 65°C.

In the step (B2), when the droplets are to be formed in the aqueous solution produced in the step (A2), the incubation temperature can be set to a temperature above the transition temperature of the peptide (P). Since the transition temperature varies depending on the amino acid sequence of the peptide (P), the incubation temperature can be set according to the transition temperature of the peptide (P). The incubation temperature can be set, for example, to a temperature 1°C to 10°C higher than the transition temperature of the peptide (P). When droplets are formed, unfolded proteins are incorporated into the droplets. On the other hand, the folded protein moves out of the droplets. As a result, by separating the droplets and the aqueous phase, the folded proteins and the unfolded proteins can be separated.

When the peptide (P-1I) and the peptide (P) that is not the peptide (P-1I) are used in combination as the peptide (P), the incubation temperature may be set to a temperature at or above the transition temperature of the peptide (P) that is not the peptide (P-II). By setting the temperature to be at or above the transition temperature of the peptide (P) that is not the peptide (P-II), liquid-liquid phase separation proceeds by the peptide (P) that is not the peptide (P-II), and thus the droplets are formed.

The incubation time may be a time sufficient for the protein to be folded. Examples of the incubation time include 5 minutes or longer, 10 minutes or longer, 15 minutes or longer, 30 minutes or longer, 45 minutes or longer, or 60 minutes or longer. The incubation time can be, for example, 5 to 180 minutes, 10 to 180 minutes, 15 to 180 minutes, 30 to 180 minutes, 45 to 180 minutes, or 60 to 180 minutes.

### (Other steps)

The protein folding method according to the present embodiment may include other steps in addition to the above-described steps (A2) and (B2). Examples of other steps include (C2) a step of recovering the folded protein from the above-described aqueous solution after the above-described step (B2).

### <<Step (C2)>>

After the step (B2), the folded protein may be recovered from the above-described aqueous solution. By the step (B2), most of the protein molecules become folded. Therefore, the folded protein can be recovered by applying a known purification method, such as reverse-phase high-performance liquid chromatography, to the aqueous solution obtained after the step (B2).

When the droplets are formed in the step (B2), the folded protein moves to the aqueous phase. Therefore, the folded protein can be recovered from the aqueous phase. Separation of the aqueous phase and the droplets can be performed by centrifugation or the like. A rotation speed of the centrifugation is, for example, 5000 to 10000 rpm. The centrifugation time includes, for example, 10 seconds or longer, and may be 20 seconds or longer. From the viewpoint of preventing destruction of the droplets, the centrifugation time is preferably 5 minutes or shorter, more preferably 3 minutes or shorter, and still more preferably 1 minute or shorter. The centrifugation time may be 10 seconds or longer and 5 minutes or shorter, 20 seconds or longer and 3 minutes or shorter, or 20 seconds or longer and 1 minute or shorter. The aqueous phase can be recovered as a supernatant of the centrifugation. After recovering the aqueous phase as the supernatant, the folded protein may be further purified using a known purification method such as reverse-phase high-performance liquid chromatography.

In the protein folding method according to the present embodiment, folding of a protein involving disulfide bond formation can be promoted with an extremely high efficiency (for example, 90% or more). Protein pharmaceuticals such as insulin and antibodies have disulfide bonds. The protein folding method according to the present embodiment can be applied to folding such proteins. Therefore, the method is expected to be applicable to the production of protein pharmaceuticals.

The peptide (P) (particularly, the peptide (P-11)) may serve as a basis for materials that promote protein quaternary structure formation. Many protein pharmaceuticals, such as antibodies and insulin, form quaternary structures composed of multiple polypeptide chains. Promoting quaternary structure formation is important for increasing the production efficiency of protein pharmaceuticals and reducing production costs. However, there are no examples of artificial materials that promote the protein quaternary structure formation. In order to promote the protein quaternary structure formation, there is a need to concentrate multiple polypeptide chains while suppressing aggregation. The aqueous two-phase system formed by the peptide (P) is effective in incorporating and concentrating denatured proteins while suppressing aggregation. In addition, using the peptide (P-II) promotes protein folding within the droplets. Accordingly, the protein folding method using the peptide (P) is expected to improve the efficiency of producing protein pharmaceuticals such as antibodies and insulin.

When the peptide (P) is composed only of natural amino acid residues, excellent biocompatibility is expected. Accumulation of denatured proteins in vivo leads to amyloid formation and causes various diseases, such as neurodegenerative diseases, type 2 diabetes, and amyotrophic lateral sclerosis (ALS). The peptide (P) is also expected to be useful as a pharmaceutical for suppressing the in vivo accumulation of denatured proteins.

### Examples

Hereinafter, the present invention will be described with reference to Experimental Examples, but the present invention is not limited to Experimental Examples.

In Experimental Examples, % by mass may be described as "wt%".

### [Experimental Example 1]

### <Preparation of denatured BPTI>

### (Reagent)

Bovine pancreatic trypsin inhibitor (BPTI) was purchased from Pro-Spec-Tany TechnoGene (Rehovot, Israel).

1,4-Dithiothreitol and urea were purchased from Nacalai Tesque, Inc. (Kyoto, Japan).

### (Experimental procedure)

BPTI (10 mg) dissolved in 0.1 M Tris-HCl (pH: 8.0, 1.0 mL) containing 30 mM 1,4-dithiothreitol and 8 M urea was incubated at 50°C for 3 hours. The fully reduced and denatured BPTI was purified by reverse-phase high-performance liquid chromatography (RP-HPLC) using a PU-4180 pump and a UV-4075 detector manufactured by JASCO Corporation (Tokyo, Japan) and an InertSustain C18 column (4.6 mmφ × 250 mm) manufactured by GL Sciences (Tokyo, Japan) at a flow rate of 1.0 mL·min-1.

Fractions of denatured BPTI were collected and freeze-dried. The obtained powder was stored at -30°C until use.

### <Synthesis of LL-ELP4>

### (Reagent)

Acetic anhydride was purchased from Kanto Chemical Co., Inc. (Tokyo, Japan).

Acetonitrile, N,N'-dimethylformamide (DMF), diethyl ether (Et2O), N-methyl-2-pyrrolidone (NMP), piperidine, and trifluoroacetic acid (TFA) were purchased from Kishida Chemical Co., Ltd. (Tokyo, Japan).

N,N-diisopropylethylamine (DIEA) was purchased from Nacalai Tesque (Kyoto, Japan).

Triisopropylsilane (TIS) was purchased from Tokyo Chemical Industry Co., Ltd. (Tokyo, Japan).

Fmoc-NH-SAL Resin, Fmoc-Gly-OH, Fmoc-Pro-OH, Fmoc-Val-OH, Fmoc-Leu-OH, 1-[bis(dimethylamino)methylene]-1H-benzotriazolium 3-oxide hexafluorophosphate (HBTU), and 1,2,3-benzotriazol-1-ol monohydrate (HOBt·H2O) were purchased from Watanabe Chemical Industries, Ltd. (Hiroshima, Japan).

Ultrapure water for HPLC (filtered through a 0.22 µm membrane filter, >18.2 MΩcm) was purified with Purelab DV35 manufactured by ELGA (Buckinghamshire, UK).

### (Experimental procedure)

LL-ELP4 (LL-(VPGVG)₄) was synthesized using a fully automatic microwave peptide synthesizer (Initiator+Alstra) from Biotage (Uppsala, Sweden) under the following conditions.
· Coupling: 75°C for 5 minutes
· Deprotection of Fmoc group: first reaction at 25°C for 3 minutes, second reaction at 25°C for 10 minutes
· N-terminal acetylation: 10 minutes at 25°C
· Washing: 4 times with DMF after coupling, 4 times with DMF after second deprotection

For the coupling reaction, deprotection reaction, and acetylation reaction, 0.5 M DMF solutions of each amino acid, HOBt, and HBTU, a 2 M NMP solution of DIEA, a 20% piperidine DMF solution, and a 5 M DMF solution of acetic anhydride were prepared.

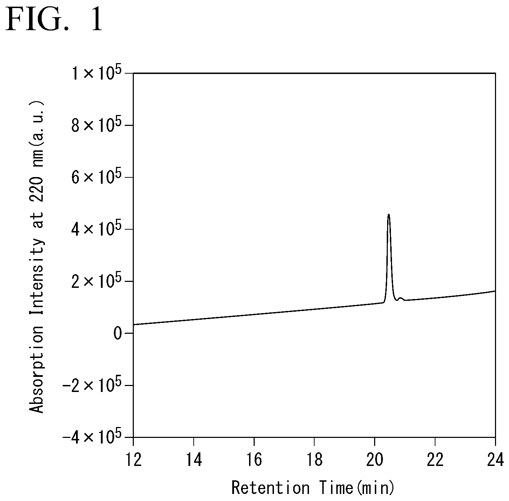

To cleave the peptide from the resin, a cleavage cocktail containing TIS (62.5 µL), TFA (2.375 mL), and water (62.5 µL) was used. The cleavage cocktail (2.5 mL) was added to the resin, and the reaction tube was left at 25°C for 90 minutes and shaken gently every 30 minutes. The mixture was filtered, and the solution was collected in a polypropylene centrifugal separator. The reaction tube was washed with TFA (500 µL, 3 times), which was also collected by filtration. Et₂O (40 mL) was added to a centrifuge tube, and the mixture was vortexed for 1 minute. Thereafter, the mixture was centrifuged at 3,500 × g and 4°C for 5 minutes using a micro refrigerated centrifuge (Model 3700) manufactured by Kubota Shoji Co., Ltd. (Tokyo, Japan), after which the supernatant was removed. After repeating the operation three times, the peptide was vacuum-dried at 25°C for 2 hours, dispersed in water, and freeze-dried using a freeze dryer (FDU-1200) manufactured by Tokyo Rikakikai Co., Ltd. (Tokyo, Japan) equipped with an oil rotary vacuum pump (GLD-051) manufactured by ULVAC, Inc. (Tokyo, Japan). The synthesized peptide was purified by performing semi-preparative reverse-phase high-performance liquid chromatography (RP-HPLC) using a PU-4086-Binary pump, a UV-4075 detector, and a CHF122SC fraction collector manufactured by JASCO Corporation (Tokyo, Japan), and a TA12S05-2520WX Actus Triart column (20 mmφ × 250 mm) manufactured by YMC Co., Ltd. (Tokyo, Japan) at a flow rate of 18.9 mL·min⁻¹ (see FIG. 1).

Gradient profile of the semi-preparative RP-HPLC: water/acetonitrile = 70/30 (0 min), 50/50 (20 min), linear gradient: 0 to 20 minutes.

The collected sample was freeze-dried and stored at -20°C. The structure of the synthesized peptide was determined by matrix-assisted laser desorption/ionization time-of-flight mass spectrometry (MALDI-TOF MS).

Device information: autoflex speed spectrometer from Bruker (Bremen, Germany), using 2,5-dihydroxybenzoic acid as a matrix, measured in reflector positive-ion mode

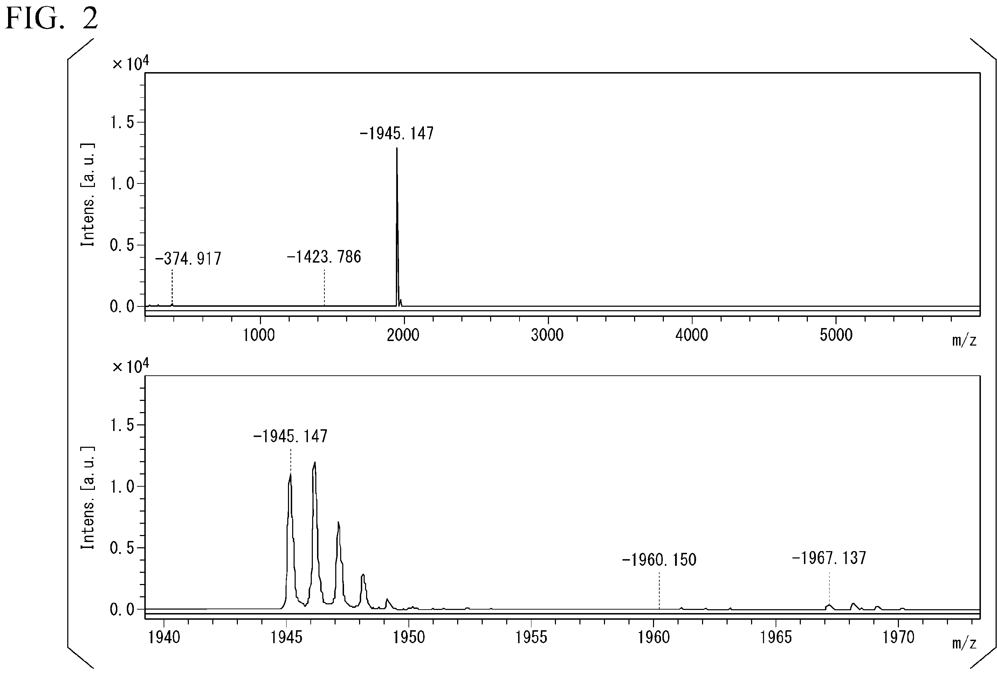

A signal of m/z 1945.147 corresponding to a complex of LL-ELP₄ and Na⁺ was exhibited (monoisotopic mass of Na⁺: 1945.125 (C₉₀H₁₅₁N₂₃O₂₃)) (see FIG. 2).

The structures of LL-ELP4 and FF-ELP4 synthesized as a control compound are shown below.

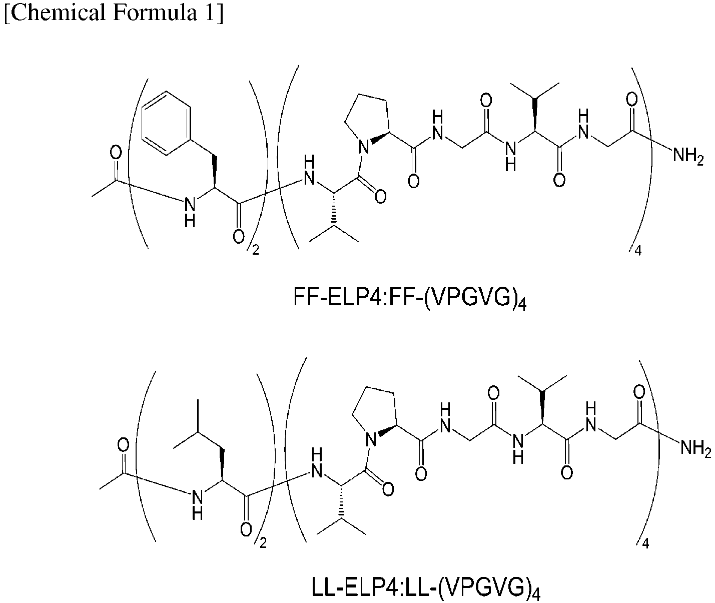

### <Transmittance measurement>

### (Experimental procedure)

FF-ELP4 or LL-ELP4 was dissolved in 50 mM Tris-HCl (pH: 7.5) containing 0.3 M sodium chloride and incubated at 60°C. LL-ELP4 was dissolved in 50 mM Tris-HCl (pH: 7.5) to a final concentration of 2 wt%. Since FF-ELP4 could not be dissolved to a final concentration of 2 wt%, it was dissolved in 50 mM Tris-HCl (pH: 7.5) to a final concentration of 1 wt%.

Transmittance measurement was carried out using an ultraviolet-visible spectrophotometer V-650 manufactured by JASCO Corporation (Tokyo, Japan). For the samples prepared as described above, absorbance at a wavelength of 600 nm was monitored while changing the temperature. A quartz cell (optical path length: 10 mm) of Sansyo Co., Ltd. was used as a measurement cell.

As an actual state observation, changes in turbidity when the temperature was changed were observed for the samples prepared as described above, and photographs were taken.

### (Result)

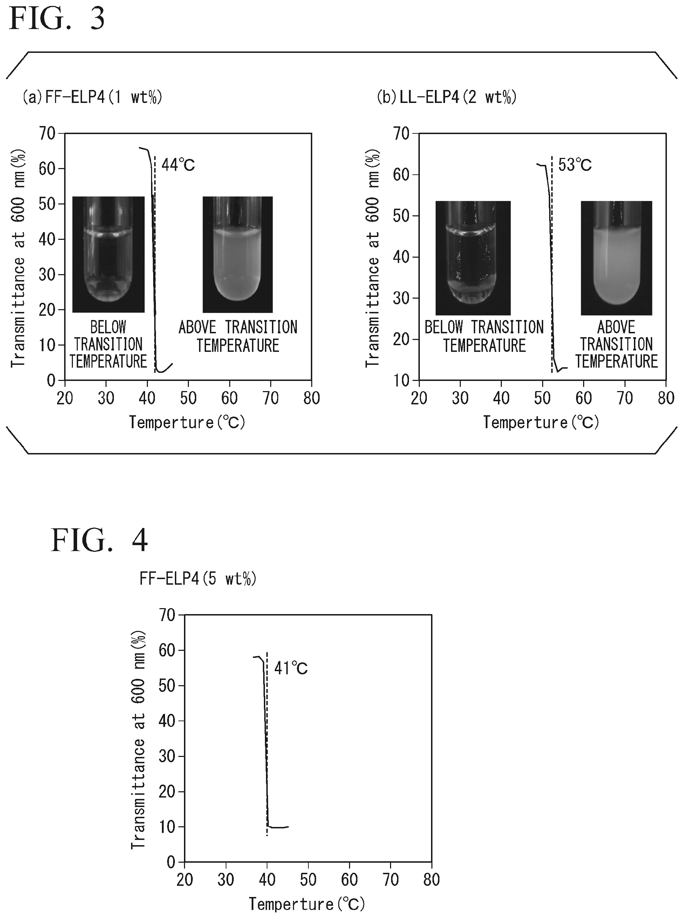

The results are shown in FIG. 3. In FIG. 3, photographs by the actual state observation are shown together in the graph showing the results of monitoring the transmittance. The transition temperature of the 1 wt% solution of FF-ELP was 44°C. The transition temperature of the 2 wt% solution of LL-ELP was 53°C.

### <Phase-contrast microscope observation>

### (Experimental procedure)

Phase-contrast microscope observation was carried out using IX-73 manufactured by Olympus Corporation (Tokyo, Japan). A sample was dropped onto a slide glass, and a cover glass was placed over the sample through a silicone spacer having a thickness of 0.2 mm. The sample was observed while incubating at 60°C. The samples were prepared by dissolving FF-ELP4 or LL-ELP4 in 50 mM Tris-HCl (pH: 7.5) containing 0.3 M sodium chloride. FF-ELP4 was dissolved to a final concentration of 1 wt%. LL-ELP4 was dissolved to a final concentration of 5 wt%.

### (Result)

FIG. 4 shows phase-contrast micrographs. In the 5 wt% solution of LL-ELP4, droplets of appropriate size were observed. On the other hand, in the 1 wt% solution of FF-ELP4, only extremely small droplets were observed.

### <Evaluation of incorporation of denatured BPTI>

### (Experimental procedure)

FF-ELP4 or LL-ELP4 was dissolved in 50 mM Tris-HCl (pH: 7.5) containing 0.3 M sodium chloride, and pre-incubated at 60°C for 1 minute. Fully reduced and denatured BPTI (170 µM) or native BPTI (170 µM) was dissolved in 1.6 M urea containing 0.05% TFA to prepare a fully reduced and denatured BPTI solution and a native BPTI solution. The fully reduced and denatured BPTI solution or the native BPTI solution was added to the pre-incubated solution (final concentration: fully reduced and denatured BPTI or native BPTI: 30 µM; urea: 70 mM; FF-ELP4: 1 wt%; LL-ELP4: 5 wt%; Tris-HCl: 50 mM; NaCl: 0.3 M, pH: 7.5). The mixture was incubated at 60°C overnight, centrifuged (8,000 rpm, 25°C, 30 seconds) using a high-spin mini centrifuge HSC-12000 manufactured by Taiyo Corporation (Osaka, Japan), and quickly separated into a supernatant and a precipitate (droplets). 400 µL of a 1 M HCl aqueous solution was added to each of the supernatant and the precipitate to stop the reaction. For each of the supernatant and the precipitate, the reduced and denatured BPTI or the native BPTI was quantified by HPLC analysis. The analysis was performed by reverse-phase high-performance liquid chromatography (RP-HPLC) using a Primaide HPLC system (1410 UV detector, 1310 column oven, 1110 pump) manufactured by Hitachi High-Tech Corporation (Tokyo, Japan) and a TSKgel Protein C4-300 column (4.6 mmφ × 150 mm) manufactured by Tosoh Corporation (Tokyo, Japan) at a flow rate of 1.0 mL·min⁻¹ (Solvent A: water containing 0.05% TFA, Solvent B: acetonitrile containing 0.05% TFA; ratio of Solvent A: linear gradient of 95% at 0 min, 80% at 15 min, and 30% at 115 min).

### (Result)

The results are shown in Table 1.

**[Table 1]**

| | | FF-ELP4 (1 wt%) | LL-ELP4 (5 wt%) | LL-ELP4 (2 wt%) |
|---|---|---|---|---|
| Reduced and denatured BPTI | Inside droplets | 17% | > 99% | 91% |
| | Outside droplets | 83% | < 1% | 9% |
| Native BPTI | Inside droplets | 6% | 7% | 3% |
| | Outside droplets | 94% | 93% | 97% |

In the 5 wt% solution of LL-ELP4, it was confirmed that the reduced and denatured BPTI was concentrated at a high concentration in the droplets. It was confirmed that the reduced and denatured BPTI recovered from the droplets could be redispersed in water and that aggregation was suppressed. On the other hand, most of the native BPTI was present outside the droplets.

In the 1 wt% solution of FF-ELP4, the reduced and denatured BPTI tended to be concentrated in the droplets. On the other hand, most of the native BPTI was present outside the droplets.

From these results, it was found that droplets formed in the LL-ELP4 solution could concentrate reduced and denatured proteins at a high concentration.

### [Experimental Example 2]

### <Preparation of denatured BPTI>

Denatured BPTI was prepared in the same manner as in Experimental Example 1.

### <Synthesis of LL-ELP4-SS>

### (Reagent)

Acetic anhydride was purchased from Kanto Chemical Co., Inc. (Tokyo, Japan).

Hydrogen peroxide, acetonitrile, N,N'-dimethylformamide (DMF), diethyl ether (Et₂O), N-methyl-2-pyrrolidone (NMP), piperidine, and trifluoroacetic acid (TFA) were purchased from Kishida Chemical Co., Ltd. (Tokyo, Japan).

N,N-diisopropylethylamine (DIEA) was purchased from Nacalai Tesque, Inc. (Kyoto, Japan).

Triisopropylsilane (TIS) was purchased from Tokyo Chemical Industry Co., Ltd. (Tokyo, Japan).

Fmoc-NH-SAL Resin, Fmoc-Gly-OH, Fmoc-Pro-OH, Fmoc-Val-OH, Fmoc-Leu-OH, 1-[bis(dimethylamino)methylene]-1H-benzotriazolium 3-oxide hexafluorophosphate (HBTU), and 1,2,3-benzotriazol-1-ol monohydrate (HOBt·H2O) were purchased from Watanabe Chemical Industries, Ltd. (Hiroshima, Japan).

Ultrapure water for HPLC (filtered through a 0.22 µm membrane filter, >18.2 MΩcm) was purified with Purelab DV35 manufactured by ELGA (Buckinghamshire, UK).

### (Experimental procedure)

LL-ELP4-SS (LL-(VPGVG)₂-CGHC-(VPGVG)₂) was synthesized using a fully automatic microwave peptide synthesizer (Initiator+Alstra) from Biotage (Uppsala, Sweden) under the following conditions.
· Coupling: 75°C for 5 minutes (here, coupling between a cysteine residue and a histidine residue was carried out at 25°C for 60 minutes)
· Deprotection of Fmoc group: first reaction at 25°C for 3 minutes, second reaction at 25°C for 10 minutes
· N-terminal acetylation: 10 minutes at 25°C
· Washing: 4 times with DMF after the coupling, 4 times with DMF after the second deprotection

For the coupling reaction, deprotection reaction, and acetylation reaction, 0.5 M DMF solutions of each amino acid, HOBt, and HBTU, a 2 M NMP solution of DIEA, a 20% piperidine DMF solution, and a 5 M DMF solution of acetic anhydride were prepared.

To cleave the peptide from the resin, a cleavage cocktail containing TIS (62.5 µL), TFA (2.375 mL), and water (62.5 µL) was used. The cleavage cocktail (2.5 mL) was added to the resin, and the reaction tube was left at 25°C for 90 minutes and shaken gently every 30 minutes. The mixture was filtered, and the solution was collected in a polypropylene centrifugal separator. The reaction tube was washed with TFA (500 µL, 3 times), which was also collected by filtration. Et₂O (40 mL) was added to a centrifuge tube, and the mixture was vortexed for 1 minute. Thereafter, the mixture was centrifuged at 3,500 × g and 4°C for 5 minutes using a micro refrigerated centrifuge (Model 3700) manufactured by Kubota Shoji Co., Ltd. (Tokyo, Japan), after which the supernatant was removed. After repeating the operation three times, the peptide was vacuum-dried at 25°C for 2 hours, dispersed in water, and freeze-dried using a freeze dryer (FDU-1200) manufactured by Tokyo Rikakikai Co., Ltd. (Tokyo, Japan) equipped with an oil rotary vacuum pump (GLD-051) manufactured by ULVAC, Inc. (Tokyo, Japan). The synthesized peptide was purified by performing semi-preparative reverse-phase high-performance liquid chromatography (RP-HPLC) using a PU-4086-Binary pump, a UV-4075 detector, and a CHF122SC fraction collector manufactured by JASCO Corporation (Tokyo, Japan), and a TA12S05-2520WX Actus Triart column (20 mmφ × 250 mm) manufactured by YMC Co., Ltd. (Tokyo, Japan) at a flow rate of 18.9 mL·min⁻¹.

Gradient profile of semi-preparative RP-HPLC: water/acetonitrile = 70/30 (0 min), 50/50 (20 min), linear gradient: 0 to 20 minutes.

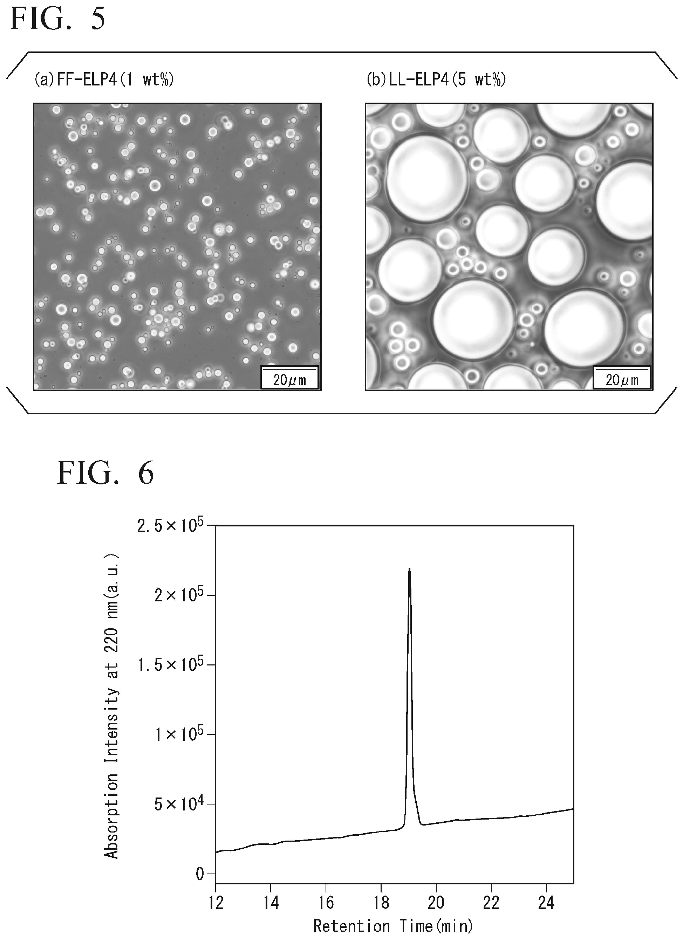

The collected sample was freeze-dried. In order to oxidize cysteine of the obtained sample, the synthesized peptide was added to water at a concentration of 1 mM and hydrogen peroxide at a concentration of 5 mM, and the mixture was stirred and reacted at room temperature under the atmosphere for 24 hours. The reaction solution was purified by performing semi-preparative reverse-phase high-performance fluid chromatography (RP-HPLC) in the same manner as after peptide synthesis (FIG. 6).

Gradient profile of semi-preparative RP-HPLC: water/acetonitrile = 70/30 (0 min), 50/50 (20 min), linear gradient: 0 to 20 minutes.

The collected sample was freeze-dried and stored at -20°C. The structure of the synthesized peptide was determined by matrix-assisted laser desorption/ionization time-of-flight mass spectrometry (MALDI-TOF MS).

Device information: autoflex speed spectrometer from Bruker (Bremen, Germany), using 2,5-dihydroxybenzoic acid as a matrix, measured in reflector positive-ion mode

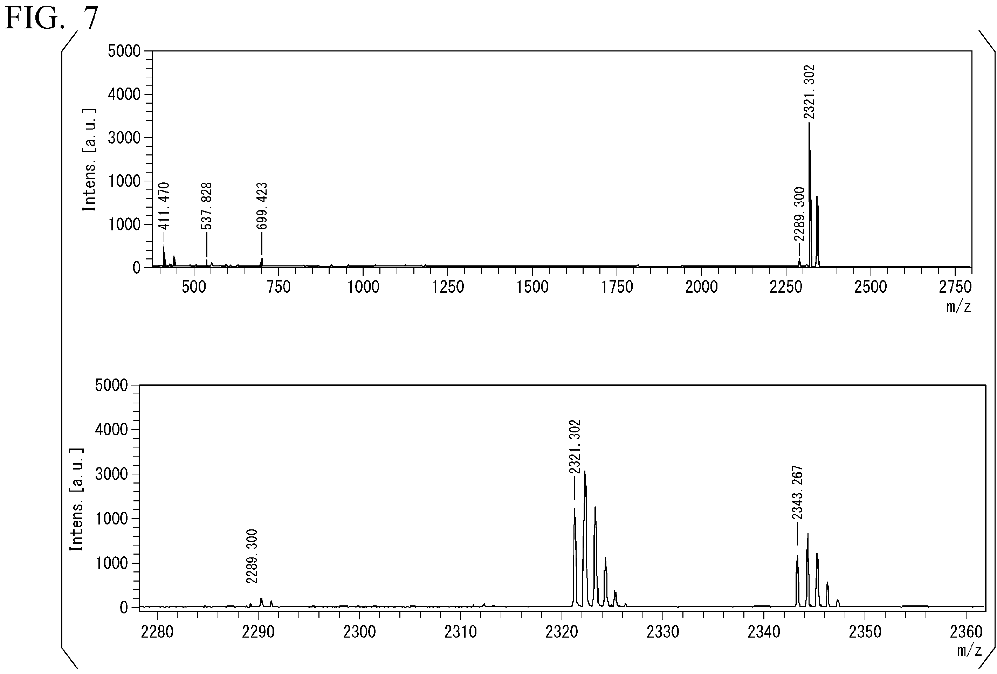

Signals of m/z 2321.302 and 2343.267 corresponding to a complex of LL-ELP4-SS and H⁺ and a complex of LL-ELP4-SS and Na⁺ were exhibited (monoisotopic mass of H⁺ and Na⁺: 2321.226, 2343.208 (C₁₀₄H₁₆₉N₂₉O₂₇S₂)) (FIG. 7).

A structure of LL-ELP4-SS is shown below.

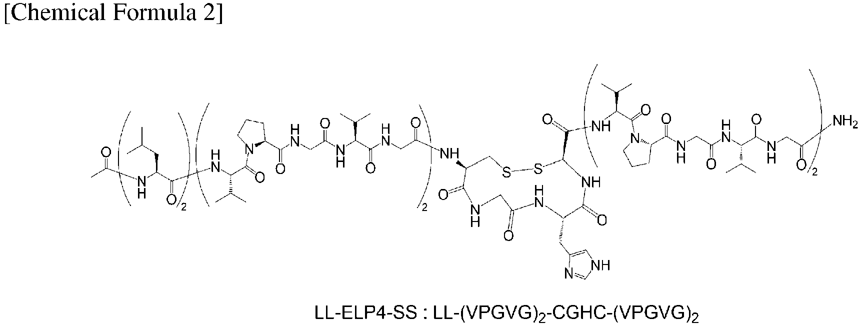

### <Transmittance measurement>

### (Experimental procedure)

LL-ELP4-SS was dissolved in 50 mM Tris-HCl (pH: 7.5) containing 0.3 M sodium chloride and incubated at 60°C. LL-ELP4-SS was dissolved in 50 mM Tris-HCl (pH: 7.5) to a final concentration of 2 wt%.

Transmittance measurement was carried out using an ultraviolet-visible spectrophotometer V-650 manufactured by JASCO Corporation (Tokyo, Japan). For the samples prepared as described above, absorbance at a wavelength of 600 nm was monitored while changing the temperature. A quartz cell (optical path length: 10 mm) of Sansyo Co., Ltd. was used as a measurement cell.

As an actual state observation, changes in turbidity when the temperature was changed were observed for the sample prepared as described above.

### (Result)

As a result of monitoring the transmittance, the transition temperature of the 2 wt% solution of LL-ELP4-SS was 48°C.

### <Phase-contrast microscope observation: LL-ELP4/LL-ELP4-SS mixed solution>

### (Experimental procedure)

Phase-contrast microscope observation was carried out using IX-73 manufactured by Olympus Corporation (Tokyo, Japan). A sample in which a cover glass was placed on the peptide solution through a silicone spacer having a thickness of 0.2 mm on a slide glass was observed while incubating at 60°C. The peptide solution was prepared by dissolving LL-ELP4 to 2 wt% and LL-ELP4-SS to 0.7 mM (0.16 wt%) in 50 mM Tris-HCl (pH: 7.5) containing 0.3 M sodium chloride.

### (Result)

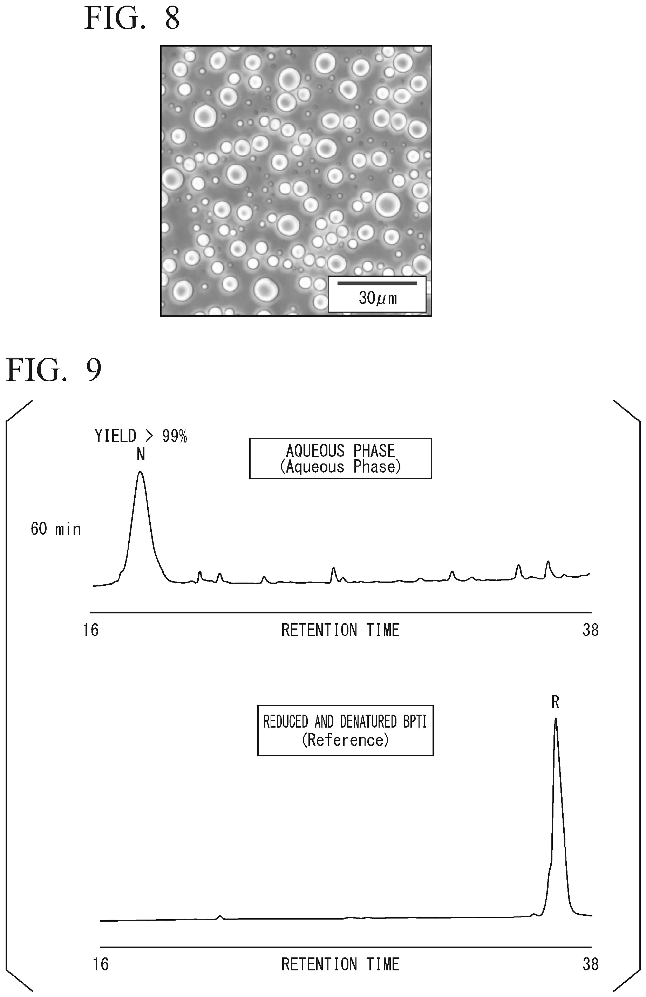

FIG. 8 shows phase-contrast micrographs. Droplets of appropriate size were observed in the peptide solution.

### <Folding test of denatured BPTI: LL-ELP4/LL-ELP4-SS mixed solution>

### (Experimental procedure)

LL-ELP4 and LL-ELP4-SS were dissolved in a buffer (50 mM Tris-HCl, 0.3 M NaCl, pH: 7.5) and pre-incubated at 60°C for 3 minutes. Fully reduced and denatured BPTI (600 µM) dissolved in 1.6 M urea containing 0.05% TFA was added to the pre-incubated solution (final concentrations: BPTI: 30 µM, urea: 80 mM, LL-ELP4: 2 wt%, LL-ELP4-SS: 0.7 mM (0.16 wt%), Tris-HCl: 50 mM, NaCl: 0.3 M, pH: 7.5). The mixture was incubated at 60°C, and when 1 minute, 10 minutes, and 60 minutes had elapsed, it was centrifuged (10,000 rpm, 25°C, 10 seconds) using a high-spin mini centrifuge HSC-12000 manufactured by Taiyo Corporation (Osaka, Japan) and quickly separated into a supernatant and a precipitate. To both the supernatant and the precipitate, 1 M aqueous HCl was added to quench the reaction. The samples were analyzed by reverse-phase high-performance liquid chromatography (RP-HPLC) using a Primaide HPLC system (1410 UV detector, 1310 column oven, and 1110 pump) manufactured by Hitachi, Ltd. (Tokyo, Japan) and a TSKgel Protein C4-300 column (4.6 mm i.d. × 150 mm) manufactured by Tosoh Corporation (Tokyo, Japan) at a flow rate of 1.0 mL min-1 (Solvent A: water containing 0.05% TFA; Solvent B: acetonitrile containing 0.05% TFA; linear gradient of Solvent A: 95% at 0 min, 80% at 15 min, and 30% at 115 min).

### (Result)

The results are shown in FIG. 9. The upper panel of FIG. 9 shows the results of chromatography of the aqueous phase (outside the droplets) of the peptide solution. "N" indicates the native structural BPTI. The lower panel of FIG. 9 shows the results of chromatography of the reduced and denatured BPTI solution as a reference. "R" indicates the reduced and denatured BPTI.

In the aqueous phase of the peptide solution, 99% or more of the added reduced and denatured BPTI was detected as the native structural BPTI. From these results, it was found that the reduced and denatured protein could be folded into a native structural protein in the mixed peptide solution of LL-ELP4 and LL-ELP4-SS. Furthermore, it was found that the native structural protein could be recovered in the aqueous phase.

### <Phase-contrast microscope observation: LL-ELP4-SS solution>

### (Experimental procedure)

Phase-contrast microscope observation was carried out using IX-73 manufactured by Olympus Corporation (Tokyo, Japan). A sample in which a cover glass was placed on the peptide solution through a silicone spacer having a thickness of 0.2 mm on a slide glass was observed while incubating at 60°C. The peptide solution was prepared by dissolving LL-ELP4-SS to 2 wt% in 50 mM Tris-HCl (pH: 7.5) containing 0.3 M sodium chloride.

### (Result)

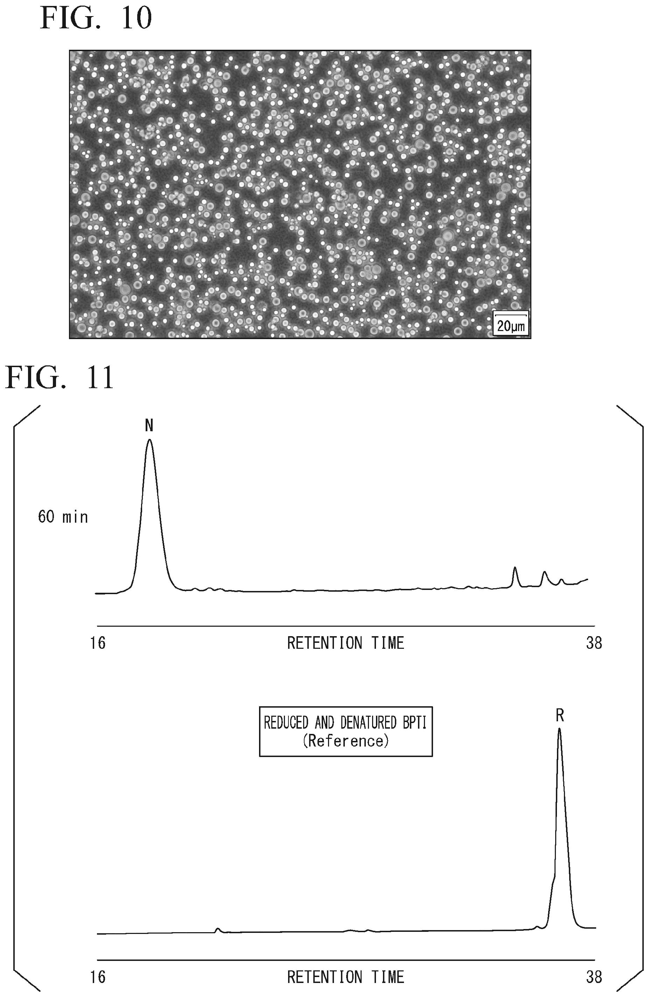

FIG. 10 shows phase-contrast micrographs. Droplets of appropriate size were observed in the peptide solution.

### <Folding test of denatured BPTI: LL-ELP4-SS solution>

### (Experimental procedure)

A folding test of denatured BPTI was performed in the same manner as in <Folding test of denatured BPTI: LL-ELP4/LL-ELP4-SS mixed solution> described above, except that LL-ELP4-SS was used alone instead of the combination of LL-ELP4 and LL-ELP4-SS. The final concentration of LL-ELP4-SS in the test solution was 0.16 wt%. In the LL-ELP4-SS solution, droplets were not formed, so the centrifugation was not carried out.

The results are shown in FIG. 11. The upper panel of FIG. 11 shows the results of chromatography of the peptide solution. "N" indicates the native structural BPTI. The lower panel of FIG. 11 shows the results of chromatography of the reduced and denatured BPTI solution as a reference. "R" indicates the reduced and denatured BPTI.

In the peptide solution, 99% or more of the added reduced and denatured BPTI was detected as native structural BPTI. From these results, it was found that the reduced and denatured protein could be folded into a native structural protein even in the peptide solution of LL-ELP4-SS alone.

From these results, it was found that folding of reduced and denatured proteins was promoted in the peptide solution containing LL-ELP4-SS. In addition, it was found that, by forming droplets using the combination of LL-ELP4-SS and LL-ELP4, the folded native structural protein could be recovered with high purity in the aqueous phase.

### INDUSTRIAL APPLICABILITY

According to the present invention, there are provided a peptide usable for protein concentration and recovery or for protein folding, a droplet forming kit containing the peptide, a droplet forming method using the peptide, a hydrophobic substance concentration method, and a protein folding method.

Hereinabove, preferable examples of the present invention have been described above, but the present invention is not limited to these examples. Configurations can be added, omitted, and replaced, and other modifications can be made within a range not departing from the gist of the present invention. The present invention is not limited by the above description, and is only limited by the scope of the appended claims.

## Claims

1. A peptide having the following characteristics (a) to (c):
(a) dissolving the peptide at an amount of 2 g or more at 20°C in 100 mL of a 50 mM Tris-HCl buffer (pH: 7.5) containing 0.3 M sodium chloride;
(b) undergoing a reversible phase transition in the Tris-HCl buffer, in which a transition temperature when 2 g of the peptide is dissolved in 100 mL of the Tris-HCl buffer is 40°C to 70°C; and
(c) forming droplets under a temperature condition at or above the transition temperature when 2 g of the peptide is dissolved in 100 mL of the Tris-HCl buffer.

2. The peptide according to Claim 1, wherein the peptide includes an amino acid sequence set forth in SEQ ID NO: 1,
VPGXG (SEQ ID NO: 1)
[in the formula, V represents a valine residue, P represents a proline residue, G represents a glycine residue, and X represents any amino acid residue other than a proline residue].

3. The peptide according to Claim 2, wherein, in addition to the amino acid sequence set forth in SEQ ID NO: 1, the peptide includes one or more hydrophobic aliphatic amino acid residues at at least one of an N-terminus and a C-terminus.

4. The peptide according to Claim 3, wherein the hydrophobic aliphatic amino acid residue is at least one selected from the group consisting of a leucine residue, an isoleucine residue, a valine residue, an alanine residue, and a methionine residue.

5. The peptide according to Claim 3, wherein the peptide includes an amino acid sequence represented by General Formula (1),
(Z1)ₙ₁-(VPGXG)ₘ-(Z2)ₙ₂ (I)
[in the formula, Z1 and Z2 each independently represent a hydrophobic aliphatic amino acid residue, V represents a valine residue, P represents a proline residue, G represents a glycine residue, and X represents any amino acid residue other than a proline residue, n1 and n2 each independently represent an integer of 0 to 3, in which n1 + n2 ≥ 1, and m represents an integer of 3 to 5].

6. The peptide according to Claim 5, wherein the amino acid sequence represented by General Formula (I) is an amino acid sequence set forth in SEQ ID NO: 2,
LL-(VPGXG)₄ (SEQ ID NO: 2)
[in the formula, L represents a leucine residue, V represents a valine residue, P represents a proline residue, G represents a glycine residue, and X represents any amino acid residue other than a proline residue].

7. The peptide according to any one of Claims 1 to 6, wherein the peptide is used for concentrating a hydrophobic substance.

8. The peptide according to Claim 7, wherein the hydrophobic substance is a denatured protein.

9. The peptide according to Claim 1, wherein the peptide includes at least one cysteine residue.

10. The peptide according to Claim 9, wherein the peptide includes an amino acid sequence represented by General Formula (II),
(Z1)ₙ₁-(Cx)ₚ₁-(VPGXG)ₘ₁-(Cy)ₚ₂-(VPGXG)ₘ₂-(Cx)ₚ₃-(Z2)ₙ₂ (II)
[in the formula, Z1 and Z2 each independently represent a hydrophobic aliphatic amino acid residue, V represents a valine residue, P represents a proline residue, G represents a glycine residue, and X represents any amino acid residue other than a proline residue, Cx, Cy, and Cz each independently represent 1 to 5 amino acid residues including at least one cysteine residue, n1 and n2 each independently represent an integer of 0 to 3, in which n1 + n2 ≥ 1, m1 and m2 each independently represent an integer of 0 to 5, in which 3 ≤ m1 + m2 ≤ 5, and p1, p2, and p3 each independently represent 0 or 1, in which p1 + p2 + p3 ≥ 1].

11. The peptide according to Claim 10, wherein the amino acid sequence represented by General Formula (II) is an amino acid sequence set forth in SEQ ID NO: 4,
LL-(VPGXG)₂-CGHC-(VPGXG)₂ (SEQ ID NO: 4)
[in the formula, L represents a leucine residue, V represents a valine residue, P represents a proline residue, G represents a glycine residue, C represents a cysteine residue, H represents a histidine residue, and X represents any amino acid residue other than a proline residue].

12. The peptide according to any one of Claims 9 to 11, wherein the peptide is used for protein folding.

13. A droplet forming kit, comprising: the peptide according to any one of Claims 1 to 6 and 9 to 11.

14. A droplet forming method, comprising:
(A) a step of producing an aqueous solution containing the peptide according to any one of Claims 1 to 6 and 9 to 11; and
(B) a step of incubating the aqueous solution at a temperature higher than a transition temperature of the peptide.

15. A hydrophobic substance concentration method, comprising:
(A1) a step of producing an aqueous solution containing the peptide according to any one of Claims 1 to 6 and a hydrophobic substance; and
(B1) a step of incubating the aqueous solution at a temperature higher than a transition temperature of the peptide.

16. The hydrophobic substance concentration method according to Claim 15, wherein the hydrophobic substance is a denatured protein.

17. A protein folding method, comprising:
(A2) a step of producing an aqueous solution containing the peptide according to any one of Claims 9 to 11 and a protein; and
(B2) a step of incubating the aqueous solution.

18. The protein folding method according to Claim 17, wherein the protein is a denatured protein.
